(19) Europäisches Patentamt
European Patent Office
Office européen des brevets

(11) Publication number : **0 509 795 A2**

# (12) EUROPEAN PATENT APPLICATION

(21) Application number : **92303401.1**

(22) Date of filing : **15.04.92**

(51) Int. Cl.[5] : **C07D 498/18,** A61K 31/395, A61K 31/71, // (C07D498/18, 311:00, 273:00, 221:00)

(30) Priority : **17.04.91 US 686728**
**18.02.92 US 837048**

(43) Date of publication of application :
**21.10.92 Bulletin 92/43**

(84) Designated Contracting States :
**AT BE CH DE DK ES FR GB GR IT LI LU NL PT SE**

(71) Applicant : **AMERICAN HOME PRODUCTS CORPORATION**
**685, Third Avenue**
**New York, New York 10017 (US)**

(72) Inventor : **Kao, Wenling**
**1742 Jennings Way**
**Paoli, Pennsylvania 91301 (US)**
Inventor : **Vogel, Robert Lewis**
**20 Sleepy Hollow Road**
**Stratford, New Jersey 08084 (US)**
Inventor : **Musser, John Henry**
**Apt. 602, Marian View Towers, Pacific Marina**
**Alameda, California 94501 (US)**

(74) Representative : **Wileman, David Francis Dr. et al**
**c/o Wyeth Laboratories Huntercombe Lane South**
**Taplow Maidenhead Berkshire SL6 OPH (GB)**

## (54) Carbamates of rapamycin.

(57) The invention concerns a compound of the structure :

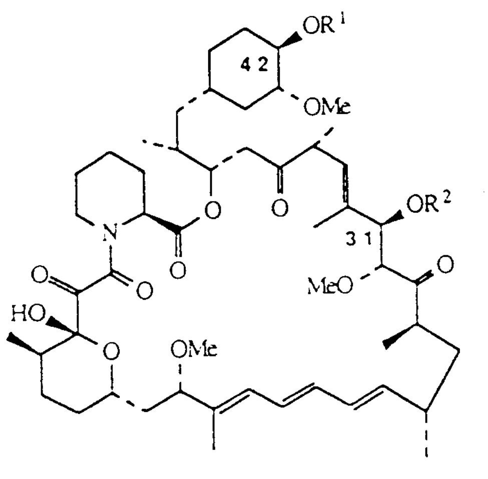

wherein $R^l$ and $R^2$ are each, independently, hydrogen, $-CONH(CR^3R^4)_n-X$ or $CONHSO_2-Ar$ ; $R^3$ and $R^4$ are each independently, hydrogen, alkyl, aralkyl, cycloalkyl of 3-8 carbon atoms, halogen, or trifluoromethyl ; providing that when more than one $R^3$ or $R^4$ group is present then they may be the same or different ; X is hydrogen, lower alkyl, cycloalkyl, trifluoromethyl, nitro, alkoxy, carboalkoxy, aralkyl, halo, dialkylamino, thioalkyl, or Y ; Y is a phenyl group which may be optionally mono-, di-, or tri- substituted ; Ar is phenyl, naphthyl, pyridyl, quinolyl, isoquinolyl, quinoxalyl, thienyl, thionaphthyl, furyl, benzofuryl, benzodioxyl, benzoxazolyl, benzoisoxazolyl, or benzodioxolyl ; wherein the Ar group may be optionally mono-, di-, or tri-subsrituted, and n = 0-5 ;
with the proviso that $R^1$ and $R^2$ are not both hydrogen and when n = 0, X is lower alkyl, cycloalkyl , aralkyl, or Y ;
or a pharmaceutically acceptable salt thereof when X is dialkylamino, when the Ar group contains a basic nitrogen, or when the Ar group is substituted by dialklyamino, $SO_3H$, $-PO_3H$, or $-CO_2H$, which by virtue of its immunosuppressive activity is useful in treating transplantation rejection, host vs. graft disease, autoimmune diseases and discases of inflammation ; by virtue of its antitumor activity is useful in treating solid tumors ; and by virtue of its antifungal activity is useful in treating fungal infections.

EP 0 509 795 A2

This invention relates to carbamates of rapamycin, processes for their preparation and pharmaceutical compositions containing them. They may be used in the treatment of transplantation rejection, host vs. graft disease, autoimmune diseases, diseases of inflammation, solid tumors and fungal infections. The invention also relates to protected carbamate derivatives of rapamycin.

Rapamycin is a macrocyclic triene antibiotic produced by Streptomyces hyproscopicus, which was found to have antifungal activity, particularly against Candida albicans. both in vitro and in vivo [C. Vezina et al., J. Antibiot. 28, 721 (1975); S.N. Sehgal et al., J. Antibiot. 28, 727 (1975); H. A. Baker et al., J. Antibiot. 31, 539 (1978); U.S. Patent 3,929,992; and U.S. Patent 3,993,749].

Rapamycin alone (U.S. Patent 4,885,171) or in combination with picibanil (U.S. Patent 4,401,653) has been shown to have antitumor activity. R. Martel et al. [Can. J. Physiol. Pharmacol. 55, 48 (1977)] disclosed that rapamycin is effective in the experimental allergic encephalomyelitis model, a model for multiple sclerosis; in the adjuvant arthriris model, a model for rheumatoid arthritis; and effectively inhibited the formation of IgE-like antibodies.

The immunosuppressive effects of rapamycin have been disclosed in FASEB 3, 3411 (1989). Cyclosporin A and FK-506, other macrocyclic molecules, also have been shown to be effective as immunosuppressive agents, therefore useful in preventing transplant rejection [FASEB 3, 3411 (1989); FASEB 3, 5256 (1989); and R. Y. Calne et al., Lancet 1183 (1978)].

Mono- and diacylated derivatives of rapamycin (esterified at the 28 and 43 positions) have been shown to be useful as antifungal agents (U.S. Patent 4,316,885) and used to make water soluble prodrugs of rapamycin (U.S. Patent 4,650,803). Recently, the numbering convention for rapamycin has been changed; therefore according to Chemical Abstracts nomenclature, the esters described above would be at the 31- and 42- positions.

This invention provides derivatives of rapamycin which are useful as immunosuppressive, anti-infammatory, antifungal, and antitumor agents having the structure

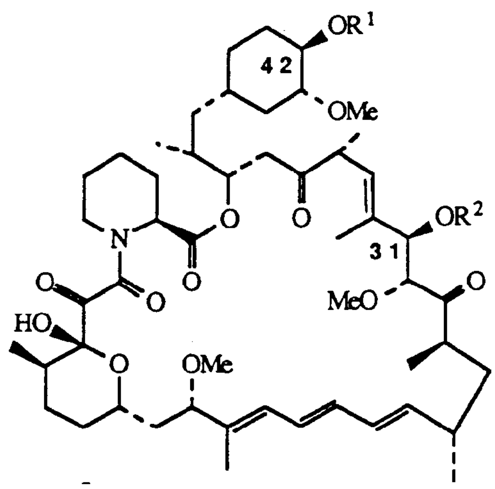

wherein $R^1$ and $R^2$ are each, independently, hydrogen, $-CONH(CR^3R^4)_n-X$ or $CONHSO_2-Ar$; $R^3$ and $R^4$ are each, independently, hydrogen, alkyl of 1-6 carbon atoms, aralkyl of 7-10 carbon atoms,cycloalkyl of 3-8 carbon atoms, halogen, or trifluoromethyl; providing that when more than one $R^3$ or $R^4$ group is present then they may each be the same or different;

X is hydrogen, lower alkyl of 1-6 carbon atoms, cycloalkyl of 3-8 carbon atoms, trifuoromethyl, nitro, alkoxy of 1-6 carbon atoms, carboalkoxy of 2-7 carbon atoms, aralkyl of 7-10 carbon atoms, halo, dialkylamino of 1-6 carbon atoms per alkyl group, thioalkyl of 1-6 carbon atoms, or Y;

Y is a phenyl group which may be optionally mono-, di-, or tri- substituted with a group selected from alkyl of 1-6 carbon atoms, aralkyl of 7-10 carbon atoms, alkoxy of 1-6 carbon atoms, cyano, halo, nitro, carbalkoxy of 2-7 carbon atoms, trifuoromethyl, dialkylamino of 1-6 carbon atoms per alkyl group, or alkylthio of 1-6 carbon atoms;

Ar is phenyl, naphthyl, pyridyl, quinolyl, isoquinolyl, quinoxalyl, thienyl, thionaphthyl, furyl, benzofuryl, benzodioxyl, benzoxazolyl, benzoisoxazolyl, or benzodioxolyl; wherein the Ar group may be optionally mono-, di-, or tri- subsbtuted with a group selected from alkyl of 1-6 carbon atoms, arylalkyl of 7-l0 carbon atoms, alkoxy of l-6 carbon atoms, cyano, halo, nitro, carbalkoxy of 2-7 carbon atoms, trifluoromethyl, amino, dialkylamino of l-6 carbon atoms per alkyl group, alkylthio of l-6 carbon atoms, $-SO_3H$, $-PO_3H$, and $-CO_2H$; and n = 0-5; with the proviso that $R^l$ and $R^2$ are not both hydrogen and when n = 0, X is lower alkyl of l-6 carbon atoms,

cycloalkyl of 3-8 carbon atoms, aralkyl of 7-I0 carbon atoms, or Y; or a pharmaceutically acceptable salt thereof when X is dialkylamino of I-6 carbon atoms per alkyl group, when the Ar group contains a basic nitrogen, or when the Ar group is substituted by dialkylamino of I-6 carbon atoms per alkyl group, $-SO_3H$,$-PO_3H$, or $-CO_2H$.

Examples of alkyl when a group or part of a group, e.g alkoxy, are methyl, ethyl, n-propyl, isopropyl and n-butyl. Examples of halogen are fluorine, chlorine and bromine. The pharmaceutically acceptable salts are those derived from such inorganic cations such as sodium, potassium, and the like; organic bases such as: mono-, di-, and trialkyl amines of I-6 carbon atoms, per alkyl group and mono-, di-, and trihydroxyalkyl amines of I-6 carbon atoms per alkyl group, and the like; and organic and inorganic acids as: acetic, lactic, citric, tartaric, succinic, maleic, malonic, gluconic, hydrochloric, hydrobromic, phosphoric, nitric, sulfuric, methanesulfonic, and similarly known acceptable acids.

Examples of n are 0, I and 2. Examples of X are benzyl, cyclohexyl, methyl, 2-propyl, trifluoromethyl, nitro, dimethylamino, carbomethoxy and methylthio, hexyl, $CHF_2$. Examples of Y are phenyl, halophenyl such as fluoro- or difluoro- phenyl; $C_I$-$C_4$ alkylphenyl such as methylphenyl; nitrophenyl and trifluormethylphenyl. Of these compounds, preferred members are those in which $R^2$ is hydrogen; those in which $R^I$ is hydrogen; those in which n is 0 and X is Y; those in which $R^2$ is hydrogen, n is 0, and X is Y; those in which n is 0, X is Y, and Y is phenyl, 4-fluorophenyl,2,4-difluorophenyl, 4-nitrophenyl, or 4-methylphenyl; and those in which Ar is optionally mono-, di-, or tri- substituted phenyl. When Ar is substituted with arylalkyl of 7-I0 carbon atoms, it is preferred that the aryl portion of the arylalkyl moiety be a phenyl group. Examples of substituents for Ar are halogen, e.g chlorine and $C_I$-$C_6$ alkyl, e.g methyl.

The compounds of this invention carbamylated at the 42-position or at both the 3I- and 42-positions can be prepared by reacting rapamycin with an isocyanate having the general structure

**O=C=N-Q**

where Q is $-(CR^3R^4)_n-X$ or $SO_2$-Ar, conveniently under neutral conditions or in the presence of a base, such as pyridine.

The 31-carbamylated compounds of this invention can be prepared by protecting the 42-alcohol of rapamycin with a protecting group, such as with a tert-butyl dimethylsilyl group, followed by carbamylation of the 31-position with an isocyanate with the general saucture shown above. Removal of the protecting group provides the 31-carbamylated compounds. In the case of the tert-butyl dimethylsilyl protecting group, deprotection can be accomplished under mildly acidic conditions.

Having the 31-position carbamylated and the 42-position deprotected, the 42-position can be reacted with a different isocyanate than was reacted with the 31-alcohol, to give compounds having different carbamates at the 31- and 42- postions. Alternatively, the 42-carbamylated compounds, prepared as described above, can be reacted with a different isocyanate to provide compounds having different carbamates at the 31- and 42-positions.

The isocyanates used to prepare the compounds of the invention are commercially available or can be prepared by methods that are disclosed in the literature. March [Advanced Organic Chemistry, 3d ed., pp. 391, 452, and 479 (1985)] describes a general method for preparing arylsulfonyl isocyanates, that can be used where the arylsulfonylisocyanate is not commercially available. The following scheme is illustrative of one method starting from an aryl moiety. Other methods of preparing arylsulfonyl isocyanates are known in the literature.

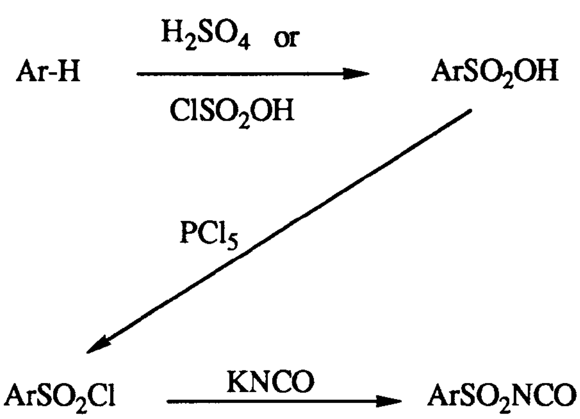


Immunosuppressive activity was evaluated in an in vitro standard pharmacological test procedure to measure lymphocyte proliferation (LAF) and in two in vivo standard pharmacological test procedures. The first in vivo procedure was a popliteal lymph node (PLN) test procedure which measured the effect of compounds of this invention on a mixed lymphocyte reaction and the second in vivo procedure evaluated the survival time of

a pinch skin graft.

The comitogen-induced thymocyte proliferation procedure (LAF) was used as an in vitro measure of the immunosuppressive effects of representative compounds. Briefy, cells from the thymus of normal BALB/c mice are cultured for 72 hours with PHA and IL-1 and pulsed with tritiated thymidine during the last six hours. Cells are cultured with and without various concentrations of rapamycin, cyclosporin A, or test compound. Cells are harvested and incorporated radioactivity is determined. Inhibition of lymphoproliferation is assessed as percent change in counts per minute from non-drug treated controls. The results are expressed by the following ratio or as an $IC_{50}$.

$$\frac{^3H-\text{control thymus cells}\ -\ H^3-\text{rapamycin}-\text{treated thymus cells}}{^3H-\text{control thymus cells}\ -\ H^3-\text{test compound}-\text{treated cells}}$$

A mixed lymphocyte reaction (MLR) occurs when lymphoid cells from generically distinct animals are combined in tissue culture. Each stimulates the other to undergo blast transformation which results in increased DNA synthesis that can be quantified by the incorporation of tritiated thymidine. Since stimulating a MLR is a function of disparity at Major Histocompatibility antigens, an in vivo popliteal lymph node (PLN) test procedure closely correlates to host vs. graft disease. Briefy, irradiated spleen cells from BALB/c donors are injected into the right hind foot pad of recipient C3H mice. The drug is given daily, p.o. from Day 0 to Day 4. On Day 3 and Day 4, tritiated thymidine is given i.p., b.i.d. On Day 5, the hind popliteal lymph nodes are removed and dissolved, and radioactivity counted. The corresponding left PLN serves as the control for the PLN from the injected hind foot. Percent suppression is calculated using the non-drug treated animals as allogenic control. Rapamycin at a dose of 6 mg/kg, p.o. gave 86% suppression, whereas cyclosporin A at the same dose gave 43% suppression. Results are expressed by the following ratio:

$$\frac{^3H-\text{PLN cells control C3H mouse}\ -\ ^3H-\text{PLN cells rapamycin}-\text{treated C3H mouse}}{^3H-\text{PLN cells control C3H mouse}\ -\ ^3H-\text{PLN cells test compound}-\text{treated C3H mouse}}$$

The second in vivo test procedure is designed to determine the survival rime of pinch skin graft from male DBA/2 donors transplanted to male BALB/c recipients. The method is adapted from Billingham R.E. and Medawar P.B., J. Exp. Biol. 28:385-402, (1951). Briefly, a pinch skin graft from the donor is grafted on the dorsum of the recipient as a homograft, and an autograft is used as control in the same region. The recipients are treated with either varying concentrations of cyclosporin A as test control or the test compound, intraperitoneally. Untreated recipients serve as rejection control. The graft is monitored daily and observations are recorded until the graft becomes dry and forms a blackened scab. This is considered as the rejection day. The mean graft survival time (number of days ± S.D.) of the drug treatment group is compared with the control group.

The following table summarizes the results of representative compounds of this invention in these three standard test procedures.

TABLE 1

| Compound | L A F IC50 (nM) | PLN (ratio) | Skin Graft (days + SD) |
|---|---|---|---|
| Example 1 | 8.6 | 0.71 | 8.5 ± 1.6 |
| Example 2 | 3.9 | 0.47 | 8.5 ± 1.2 |
| Example 3 | >1000 | + | + |
| Example 4 | 57.9 | 0.90 | + |
| Example 5 | 3%++ | + | + |
| Example 6 | 10.4 | 0.25 | 8.3 ± 1.0 |
| Example 7 | + | + | + |
| Example 8 | 7.4 | + | 8.8 ± 1.3 |
| Example 9 | 8.7 | 0.72 | 8.0 ± 1.7 |
| Example 10 | >1000 | + | + |
| Example 11 | >1000 | + | + |
| Example 12 | >1000 | + | + |
| Example 14 | >1000 | + | + |
| Example 15 | >1000 | + | + |
| Example 16 | 0.91 (ratio) | 0.84 (p.o.)<br>1.03 (i.p.) | 11.2 ± 0.4 |
| Example 18 | 0.74 (ratio) | 0.47 | + |
| Example 19 | 0.10 (ratio) | + | + |
| Rapamycin | 3.2-9.4 | 1.00 | 12.0 ± 1.7 |

\+ Not evaluated.

++ Percent inhibition at 100 nM.

The results of these standard pharmacological test procedures demonstrate immunosuppressive activity both in vitro, and in vivo, for the compounds of this invention. Positive ratios in the LAF and PLN test procedures indicate suppression of T cell proliferation. As a transplanted pinch skin grafts are typically rejected within 6-7 days without the use of an immunosuppressive agent, the increased survival time of the skin graft when treated with the compounds of this invention further demonstrates their utility as immunosuppressive agents.

The ability of the compounds of this invention to prevent or treat transplantation was demonstrated in a heterotropic heart allograft standard pharmacological test procedure that emulates transplantation rejection that occurs in humans. The following briefly describes the procedure that was used. Male BN rat neonate donors (less than 5 days of age) were humanely sacrificed, the thymus was dissected away from the heart. All connections with the thoracic cavity were severed and the heart was removed from the chest cavity and placed in cooled RPMI media where all adherent fat and fascia were removed. The heart was bisected in half, along the midline from the apex to the root of the aorta, to generate two approximately equal halves each containing atrial and ventricular tissue. Recipient male Lewis rats were anesthetized with phenobarbitol (50 mg/mL; i.p.), the left inner ear was swabbed with povidine iodine, and 1 mL RPMI was injected subcutaneously above the cartiledge plate to produce a fluid filled sac. A stab incision was made to the sac, into which was inserted a single half heart fragment. The pocket was sealed with a single drop of Vet-Seal (3M Animal Care Products). Recipients were divided into treatment groups of 10 rats each and the compounds to be evaluated were administered at a dosage of 225 μg/day following the transplantation procedure until graft failure occurred. Administration

was i.p., either by manual injection or via an Azlet osmoric pump that was implanted into the peritoneum of the recipient rat. Grafts were inspected for loss of cardiac activity on day 7 post-transplant and subsequently on alternate days. Graft survival time is defined as the post-transplant day on which the heart graft has lost all contractile activity by visual inspection and/or cardiac monitor. Individual rejection times were averaged to produce a mean survival time for each treated group. The following table shows the results obtained for a representative compound of this invention. The control group represents rats that received the allograft and no test compound. Rapamycin was evaluated for the purpose of comparison.

TABLE 2

| Treatment Group | Mean Survival Time (days) |
|---|---|
| Control | 11.5 |
| Example 16 | 34.4 |
| Rapamycin | 38 |

As shown in Table 2, treatment with a representative compound of this invention significantly ($p < 0.05$) prolonged survival time of the transplanted allograft. The untreated allograft was rejected by 11.5 days, whereas treatment with a representative compound of this invention prevented rejection until 34.4 days. As such, the results of this standard pharmacological test procedure demonstrate that the compounds of this invention are useful in preventing or treating transplantation rejection.

Because the compounds of this invention are structurally similar to rapamycin and have a similar activity profile to rapamycin, the compounds of this invention also are considered to have antitumor and antifungal activities.

Based on the results of these standard pharmacological test procedures, the compounds are useful in the treatment of transplantation rejection such as, heart, kidney, liver, bone marrow, and skin transplants; autoimmune diseases such as, lupus, rheumatoid arthriris, diabetes mellitus, myasthenia gravis, and multiple sclerosis; and diseases of inflammation such as, psoriasis, dermatitis, eczema, seborrhea, inflammatory bowel disease, and eye uveitis; solid tumors; and fungal infections.

As the compound of Example 18 was prepared via its 42-silylated intermediate (Example 17), the compound of Example 17 is therefore a useful as an intermediate of compounds of this invention.

The compounds may be administered neat or with a pharmaceutical carrier to a mammal in need thereof. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, favoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellent.

Liquid pharmaceutical compositions which are sterile solutions or suspensions can be utilized by, for example, intramuscular, intraperitoneal or subcutaneous injection. Sterile solutions can also be administered intravenously. The compound can also be administered orally either in liquid or solid composition form.

Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form. The dosage to be used in the treatment must be subjectively determined by the attending physician.

In addition, the compounds of this invention may be employed as a solution, cream, or lotion by formulation with pharmaceutically acceptable vehicles containing 0.1 - 5 percent, preferably 2%, of active compound which may be administered to a fungally affected area.

The following examples illustrate the preparation of representative compounds of this invention.

**Example 1**

Rapamycin 42-ester with (4-fluorophenyl)carbamic acid

A solution of 70 mg of rapamycin in 1 mL of pyridine was treated at 0° under anhydrous conditions with 50 mg of parafluorophenyl isocyanate in 1.5 ml of pyridine. After stirring at 0° for 5 hours, the reaction mixture was diluted with 20 ml of 2N HCl at 0° and extracted with ethyl acetate. The ethyl acetate extract was washed with brine, dried with magnesium sulfate and evaporated. The residue was chromatographed on silica gel. Elution with 30% ethyl acetate in benzene afforded 15 mg of the title compound as a white solid, mp 99- 103°.
IR = KBr max 3440 (OH), 1740 (C=0), 1625 (amide C=0), 1460, 1855, 1200, 1070, 990 $cm^{-1}$. $^1H$ NMR ($CDCl_3$, 400 MHz), δ 7.38 (t, 2H, aromatic protons, ortho to fluorine), 7.00 (t, 2H, aromatic protons meta to fuorine), 3.39 (s, 3H, $CH_3O$), 3.33 (s, 3H, $CH_3O$); 3.14 (s, 3H $CH_3O$). MS (neg. ion FAB) 1050 ($M^-$), 590, 458, 167.

**Example 2**

Rapamycin 42-ester with phenylcarbamic acid

A solution of 100 mg of rapamycin in 0.8 ml of pyridine was treated at 0° under anhydrous conditions with 60 mg of phenyl isocyanate in 0.5 ml of pyridine. After stirring at 0° anhydrous for 5 hours, the reaction mixture was diluted with 20 ml of 2N HCl at 0°and extracted with ethyl acetate. The ethyl acetate extract was washed with brine, dried with magnesium sulfate and evaporated. The residue was chromatographed on silica gel. Elution with 25% ethyl acetate in benzene afforded 65 mg of the title compound as a white solid, mp 106-109°.
IR = KBr max 3400 (OH), 1725 (C=0), 1645 (amide C=0), 1600 (aromatic) 1540, 1440, 1315, 1080, 990 and 750 (aromatic). $^1H$ NMR ($CDCl_3$, 400 MHz), δ 7.40 (t, 2H, aromatic, ortho to NH), 7.32 (t, 2H, aromatic, 3.34 (s, 3H, $CH_3O$), 3.14 (s, 3H, $CH_3O$). MS (neg. ion FAB) 1032 ($M^-$), 590, 321, 167.

**Example 3**

Rapamycin-31,42-diester with [4-(trifluoromethyl)phenyl]carbamic acid

a) A solution of 0.45g 4-(trifluoromethyl)phenylisocyanate in 5 mL dry pyridine was added to a solution of 1.00g rapamycin in 10 mL dry pyridine at 0°C, and the resulting solution was stirred at 0-5°C for 3.5 hours. Cold 2N HCl (75 mL) was added and the product was extracted into ethyl acetate, which was washed with brine, dried over magnesium sulfate and evaporated to dryness. The residue was chromatographed on silica gel. Elution with 10% ethyl acetate in methylene chloride afforded 0.63 g of 31,42-diester as a white solid, m.p. 147-195°C IR(KBr): 3400 (OH), 1730 (C=0), 1615, 1530, 1320,1210, 1110, 1060 and 840 $cm^{-1}$. $^1H$ NMR ($CDCl_3$, 400 MHz): 7.46-7.57 (complex, 8H, aromatic), 3.38 (s, 3H, $OCH_3$), 3.36 (s, 3H, $OCH_3$), 3.16 (s, 3H, $OCH_3$), MS (neg ion FAB): 1287 ($M^-$), 1081, 894, 590, 321.

b) The following representative compounds can be prepared from rapamycin and the appropriate isocyanate by employing the method used to prepare the title compound in Example 3.

Rapamycin-31,42-diester with (phenylmethyl)carbamic acid
Rapamycin-31,42-diester with cyclohexylcarbamic acid
Rapamycin-31,42-diester with methylcarbamic acid
Rapamycin-31,42-diester with 2-propylcarbamic acid
Rapamycin-31,42-diester with trifluoromethylcarbamic acid
Rapamycin-31,42-diester with 2-nitroethylcarbamic acid
Rapamycin-31,42-diester with [4(N,N-dimethyl)butyl]carbamic acid

Rapamycin-31,42-diester with [2-(carbomethoxy)ethyl]carbamic acid
Rapamycin-31,42-diester with [4-(methylthio)butyl]carbamic acid
Rapamycin-31,42-diester with [3-ethyl-4-(4-nitrophenyl)butylcarbamic acid

**Example 4**

Rapamycin-42-ester with [4-(trifluoromethyl)phenyl]carbamic acid

a) Continued elution of the residue described in Example 3 with 30% ethyl acetate in methylene chloride afforded 0.20g 42-monoester as a white solid, mp 144-172° IR (KBr): 3450 (OH), 1730 (C=0), 1650, 1615, 1535, 1445, 1315, 1210, 1110, 1050, 980 and 835 $cm^{-1}$. $^1H$ NMR ($CDCl_3$, 400 MHz): δ 7.55 (d, 2H, aromatic), 7.50 (d, 2H, aromatic), 3.39 (s, 3H, $OCH_3$), 3.34 (s, 3H, $OCH_3$), 3.14 (s, 3H, $OCH_3$). MS (neg ion FAB): 1100 ($M^-$), 590, 321, 160.

b) The following representative compounds can be prepared from rapamycin and the appropriate isocyanate by employing the method used to prepare the title compound in Example 4.

Rapamycin-42-ester with (phenylmethyl)carbamic acid
Rapamycin-42-ester with cyclohexylcarbamic acid
Rapamycin-42-ester with methylcarbamic acid
Rapamycin-42-ester with 2-propylcarbamic acid
Rapamycin-42-ester with trifuoromethylcarbamic acid
Rapamycin-42-ester with 2-nitroethylcarbamic acid
Rapamycin-42-ester with [4-(N,N-dimethyl)butyl]carbamic acid
Rapamycin-42-ester with [2-(carbomethoxy)ethyl]carbamic acid
Rapamycin-42-ester with [4-(methylthio)butyl]carbamic acid
Rapamycin-42-ester with [3-ethyl-4-(4-nitrophenyl)butylcarbamic acid

**Example 5**

Rapamycin-31,42-diester with (4-nitrophenyl)carbamic acid

A solution of 0.38g 4-nitrophenylisocyanate in 4 mL pyridine was added to a solution of 1.16 g rapamycin in 11 mL pyridine at 0° and stirred at 0° for 15 minutes, then warmed to 20° over 15 minutes and stirred at 20° for 1.5 hours. Cold 2N HCl (75 mL) was added, the products were extracted into ethyl acetate, washed with brine, dried over $MgSO_4$ and evaporated to dryness. The residue was chromatographed on silica gel; elution with 10% ethyl acetate in methylene chloride afforded 160 mg of the diester as a yellow solid, mp 164-171°. IR (KBr): 3380 (OH), 2910, 1725 (C=0), 1635, 1590, 1500, 1320, 1200 and 850 $cm^{-1}$. $^1H$ NMR ($CDCl_3$, 400 MHz): δ 8.20 (d, 4H, aromatic), 7.57 (d, 2H, aromatic), 7.53 (d, 2H, aromatic), 3.38 (s, 3H, $OCH_3$), 3.37 (s, 3H, $OCH_3$), 3.16 (s, 3H, $OCH_3$). MS (neg ion FAB): 1241 ($M^-$),1058, 590.

**Example 6**

Rapamycin-42-ester with (4-nitrophenyl)carbamic acid

Further elution of the residue described in Example 5 with 30% ethyl acetate in methylene chloride afforded 380 mg of the 42-monoester as a yellow solid, mp 137-144°. IR (KBr): 3420 (OH), 2910, 1725 (C=0), 1500, 1320, 1205 and 840 $cm^{-1}$. NMR ($CDCl_3$, 400 MHz): δ 8.20 (d, 2H, aromatic), 7.56 (d, 2H, aromatic), 3.39 (s, 3H, $OCH_3$), 3.34 (s, 3H, $OCH_3$), 3.14 (s, 3H, $OCH_3$). MS (neg ion FAB): 1077 ($M^-$), 590, 321.

**Example 7**

Rapamycin-31,42-diester with (4-methylphenyl)carbamic acid

A solution of 1.04g 4-methylphenylisocyanate in 3 mL pyridine was added to a solution of 2.20g rapamycin in 8 mL pyridine at 0° and stirred at 0° for 3.5 hours. Cold 2N HCl (180 mL) was added, the products were extracted into ethyl acetate, washed with brine, dried over $MgSO_4$ and evaporated to dryness. The reside was chromatographed on silica gel; elution with 10% ethyl acetate in methylene chloride afforded 300 mg of 31,42-diester as a yellow solid, mp 129-145°.

IR(KBr): 3400 (OH), 2920, 1725 (C=0), 1645, 1520, 1450, 1215, 1200 and 812 $cm^{-1}$. NMR ($CDCl_3$, 400 Mhz):

δ 7.27 (complex, 4H, aromatic), 7.10 (complex, 4H, aromatic), 3.38 (s, 3H, $OCH_3$), 3.35 (s, 3H, $OCH_3$), 3.16 (s, 3H, $OCH_3$), 2.30 (s, 3H, $ArCH_3$), 2.28 (s, 3H, $ArCH_3$). MS (neg ion FAB): 1179 ($M^-$), 1027.

**Example 8**

Rapamycin-42-ester with (4-methylphenyl)carbamic acid

Further elution of the residue described in Example 7 with 30% ethyl acetate in methylene chloride afforded 200 mg of the 42-monoester as a yellow solid, mp 123-135°. IR (KBr): 3420 (OH), 2930, 1720 (C=0), 1640, 1520, 1440, 1220, 1203, and 1055 $cm^{-1}$. NMR ($CDCl_3$, 400 MHz): δ 7.27 (d, 2H, aromatic), 7.10 (d, 2H, aromatic), 3.40 (s, 3H, $OCH_3$), 3.34 (s, 3H, $OCH_3$), 3.14 (s, 3H, $OCH_3$), 2.30 (s, 3H, $ArCH_3$). MS (neg ion FAB): 1046 ($M^-$), 590, 321.

**Example 9**

Rapamycin-42-ester with (2,4-difuorophenyl)carbamic acid

A solution of 0.24g rapamycin and 0.10g 2,4-difluorophenylisocyanate in 6 mL pyridine was stirred for 45 minutes at 0°. Cold 2N HCl (50 mL) was added, the product was extracted into ethyl acetate, washed with brine, dried over $MgSO_4$ and evaporated to dryness. Chromatography on silica gel using 25% ethyl acetate in methylene chloride afforded 69 mg of 42-ester as a white solid, mp 109-114° . IR (KBr): 3420 (OH), 2910, 1720 (C=0), 1635, 1520, 1215, 1085 and 975 $cm^{-1}$. NMR ($CDCl_3$, 400 MHz): δ 6.84 (complex, 2H, aromatic), 6.69 (complex, 1H, aromatic), 3.39 (s, 3H, $OCH_3$), 3.33 (s, 3H, $OCH_3$), 3.14 (s, 3H, $OCH_3$). MS (neg ion FAB): 1068 ($M^-$), 590, 321.

**Example 10**

Rapamycin-31,42-diester with (2.4-difluorophenyl)carbamic acid

A solution of 0.20g rapamycin and 0.14g 2,4-difluorophenylisocyanate in 10 mL pyridine was stirred 5 hours at 20°. Cold 2N HCl (50 mL) was added, the product was extracted into ethyl acetate, washed with brine and dried over $MgSO_4$. The solvent was evaporated off and the residue chromatographed on silica gel using 10% ethyl acetate in methylene chloride yielded 0.20g of title compound as a pale yellow solid, mp 115-123°. IR (KBr): 3420 (OH), 2910, 1725 (C=0), 1638, 1523, 1212, 1090 and 835 $cm^{-1}$. NMR ($CDCl_3$, 400 MHz): δ 6.85 (complex, 4H, aromatic), 6.70 (complex, 2H, aromatic), 3.39 (s, 3H, $OCH_3$), 3.36 (s, 3H, $OCH_3$), 3.15 (s, 3H, $OCH_3$). MS: (neg ion FAB): 1223 ($M^-$), 1049, 590, 321.

**Example 11**

Rapamycin-31,42-diester with phenylcarbamic acid

A solution of 0.50g rapamycin and 0.19g phenylisocyanate in 10 mL pyridine was stirred at 20° for 16 hours. Cold 2N HCl (100 mL) was added and the product was extracted into ethyl acetate, washed with brine and dried over $MgSO_4$. The solvent was removed and the residue chromatographed on silica gel using 10% ethyl acetate in methylene chloride, yielded 0.32g title compound as a white solid, mp 139-148°. IR(KBr): 3330 (OH), 2920, 1720 (C=0), 1670, 1590, 1520, 1430, 1205, 745 and 685 $cm^{-1}$. NMR ($CDCl_3$, 400 MHz): δ 7.27-7.40 (complex, 8H, aromatic), 7.06 (complex, 2H, aromatic), 3.39 (s, 3H, $OCH_3$), 3.37 (s, 3H, $OCH_3$), 3.16 (s, 3H, $OCH_3$). MS (neg ion FAB): 1151 ($M^-$), 1013, 590, 321.

**Example 12**

Rapamycin-31,42-diester with (4-fluorophenyl)carbamic acid

A solution of 1.54g rapamycin and 0.68g 4-fluorophenylisocyanate in 10 mL pyridine was stirred at 0° for 4.5 hours. Cold 2N HCl (100 mL) was added, the product was extracted into ethyl acetate, washed with brine, dried over $MgSO_4$, evaporated to dryness, and chromatographed on silica gel using 10% ethyl acetate in methylene to afford 0.65g product as a white solid, mp 129-140°. IR (KBr): 3430 (OH), 2920, 1725 (C=0), 1640, 1505, 1200 and 825 $cm^{-1}$. NMR ($CDCl_3$, 400 MHz): δ 7.33 (complex, 4H, aromatic), 6.99 (t, 4H, aromatic), 3.38

(s, 3H, $OCH_3$), 3.35 (s, 3H, $OCH_3$), 3.16 (s, 3H, $OCH_3$). MS (neg ion FAB): 1187 ($M^-$), 1031, 590, 321.

**Example 13**

Rapamycin-42-tert-butyldimethylsilyl ether

Rapamycin (0.914g) was added to a solution of 0.204 g imidazole and 0.165 g tert-butyldimethylsilyl chloride in 4 mL dimethylformamide at 0° and stirred under nitrogen for 16 hours at 20°. Brine (100 mL) was added, the product was extracted into ether, washed with brine, dried over $MgSO_4$ and evaporated. Chromatography on silica gel afforded 0.65g of the title compound as a white solid. IR(KBr): 3430 (OH), 2920, 1715 (C=0), 1645, 1105, 985, 870, 842 and 775 $cm^{-1}$. NMR ($CDCl_3$, 400 MHz): $\delta$ 3.41 (s, 3H, $OCH_3$), 3.34 (s, 3H, $OCH_3$), 3.14 (s, 3H, $OCH_3$), 0.886 (s, 9H, t-Bu), 0.074 (s, 3H, $SiCH_3$), 0.059 (s, 3H, $SiCH_3$). MS (neg ion FAB): 1027 ($M^-$),590, 435.

**Example 14**

Rapamycin-42-tert-butyldimethylsilyl ether-31-ester with (2,4-difluorophenyl)-carbamic acid.

a) A solution of 0.38g of rapamycin-42-tert-butyl-dimethylsilyl ether and (2,4-difluorophenyl)isocyanate in 6 mL pyridine was stirred at 20 for 16 hours. Cold 2N HCl (75 mL) was added, the product was extracted into ethyl acetate, washed with brine, dried over $MgSO_4$, evaporated and the residue chromatographed on silica gel using 2% ethyl acetate in methylene chloride to afford 150 mg of title compound as a white solid, mp 112-122°. IR(KBr): 3440 (OH), 2910, 1745 (C=0), 1640, 1520, 1090, 840 and 765 $cm^{-1}$. NMR ($CDCl_3$, 400 MHz): $\delta$ 6.84 (complex, 3H, aromatic), 3.41 (s, 3H, $OCH_3$), 3.36 (s, 3H, $OCH_3$), 3.15 (s, 3H, $0CH_3$), 0.88 (pH, t-butyl), 0.075 (3H, $SiCH_3$), 0.061 (3H, $SiCH_3$). MS (neg ion FAB): 1182 ($M^-$), 1027, 1008, 590, 435.

b) The following representative compounds can be prepared from rapamycin and the appropriate isocyanate by employing the method used to prepare the title compound in Example 14.

Rapamycin-42-tert-butyldimethylsilyl ether-31-ester with (phenylmethyl)carbamic acid
Rapamycin-42-tert-butyldimethylsilyl ether-31-ester with hexylcarbamic acid
Rapamycin-42-tert-butyldimethylsilyl ether-31-ester with difluoromethylcarbamic acid
Rapamycin-42-tert-butyldimethylsilyl ether-31-ester with [4-(N,N-dimethyl)butyl]-carbamic acid
Rapamycin-42-tert-butyldimethylsilyl ether-31-ester with [4-(carbomethoxy)butyl]-carbamic acid
Rapamycin-42-tert-butyldimethylsilyl ether-31-ester with [2-(phenylmethyl)butyl]-carbamic acid

**Example 15**

Rapamycin-31-ester with (2,4-difluorophenyl)carbamic acid

a) Rapamycin-42-tert-butyldimethylsilyl ether-31-ester with (2,4-difluorphenyl)carbamic acid (0.60 g) was stirred for 16 hours at 20° in a solution of 4 mL tetrahydrofuran, 4 mL water and 12 mL acetic acid. After dilution with 100 mL water, the product was extracted into methylene chloride, washed with brine, dried over $MgSO_4$ and evaporated to an oil, which was chromatographed on silica gel using 30% ethyl acetate in methylene chloride to afford 220 mg of title compound as a white solid, mp 103-117° . IR (KBr): 3440, 2920, 1735 (C=0), 1640, 1525, 1090 and 840 $cm^{-1}$. NMR ($CDCl_3$, 400 MHz): $\delta$ 6.85 (complex, 3H, aromatic), 3.40 (s, 3H, $OCH_3$), 3.36 (s, 3H, $OCH_3$), 3.15 (s, 3H, $OCH_3$). MS (neg ion FAB): 1068 ($M^-$), 894, 590, 128.

b) The following representative compounds can be prepared from rapamycin and the appropriate isocyanate by employing the method used to prepare the title compound in Example 15.

Rapamycin-31-ester with (phenylmethyl)carbamic acid
Rapaycin-31-ester with hexylcarbamic acid
Rapamycin-31-ester with difuoromethylcarbamic acid
Rapamycin-31-ester with [4-(N,N-dimethyl)butyl]carbamic acid
Rapamycin-31-ester with [4-(carbomethoxy)butyl]carbamic acid
Rapamycin-31-ester with [2-(phenylmethyl)butyl]carbamic acid

**Example 16**

Rapamycin 42-ester with phenylsulfonylcarbamic acid

A solution of 192 mg of rapamycin in 1 mL toluene was treated at 0° under nitrogen with 37 mg of benzenesulfonyl isocyanate in 1 mL toluene. After stirring at 0° under nitrogen for one hour, the reaction mixture was diluted with 100 ml of ethyl acetate and washed with brine (3 x 20 ml). The ethyl acetate solution was dried with magnesium sulfate and evaporated. The residue was chromatographed on silica gel. Elution with ethyl acetate/hexane (1:1) afforded 36 mg of the title compound as a white foam.
IR (KBr) 3440 (OH), 2910, 1735 & 1710 (C=O), 1635 (aromatic), 1440, 1160, 1080 and 980 $cm^{-1}$. NMR ($CDCl_3$, 400 MHz): δ 8.04 (m, 2H, aromatic), 7.65 (m, 1H, aromatic), 7.56 (m, 2H, aromatic), 3.41 (s, 3H, $OCH_3$), 3.34 (s, 3H, $OCH_3$), 3.13 (s, 3H, $OCH_3$). MS (neg. ion FAB): 1095 ($M^-$), 590. 321.

**Example 17**

Rapamycin 42-tertbutyldimethylsilyl ether-31-ester with phenyl sulfonyl carbamic acid

a) Rapamycin (0.914g) was added to a solution of 0.204 g imidazole and 0.165 g tert-butyldimethylsilyl chloride in 4 mL dimethylformamide at 0° and stirred under nitrogen for 16 hours at 20°. Brine (100 mL) was added, the product was extracted into ether, washed with brine, dried over $MgSO_4$ and evaporated. Chromatography on silica gel afforded 0.65g of rapamycin-42-tert-butyldimethylsilyl ether as a white solid.
b) A solution of 308 mg of rapamycin-42-tert-butyldimethylsilyl ether in 1 mL toluene was treated at 0° under nitrogen with 109 mg of benezenesulfonyl isocyanate in 1 mL toluene. After stirring at 0° under nitrogen for three hours, the reaction mixture was diluted with 100 mL ethyl acetate and washed with brine (2 x 20 ml). The ethyl acetate solution was dried with magnesium sulfate and evaporated. The residue was chromatographed on silica gel. Elution with ethyl acetate/hexane (1:1) afforded 310 mg of the title compound as a white foam.
IR (KBr): 3400 and 3240 (OH), 2915, 1740 and 1710 (C = O), 1640, 1440, 1160, 1080 $cm^{-1}$. NMR ($CDCl_3$, 400 MHz): δ 8.00 (m, 2H, aromatic), 7.55 (m, 3H, aromatic), 3.45 (s, 3H, $OCH_3$), 3.27 (s, 3H, $OCH_3$), 3.13 (s, 3H, $OCH_3$). MS (neg. ion FAB): 1209 ($M^-$), 590, 156.

**Example 18**

Rapamycin-31-ester with phenylsulfonylcarbamic acid

A solution of 250 mg of Rapamycin-42-tert-butyldimethylsilyl ether-31 -ester with phenylsulfonylcarbamic acid in 2 mLof tetrahydrofuran was treated with 2 mL water and 6 mL acetic acid. After stirring at room temperature under nitrogen for 20 hours, the mixture was diluted with 130 mL ethyl acetate and washed with brine (2 x 20 ml). The ethyl acetate solution was dried with magnesium sulfate and evaporated to dryness to give 208 mg of the title compound as a white foam.
IR (KBr): 3400 (OH), 2930, 1730 (C=O), 1640 (aromatic), 1440, 1340, 1160, 1085 $cm^{-1}$' NMR ($CDCl_3$, 400 MHz): δ 8.0 (m, 2H, aromatic), 7.59 (m, 3H, aromatic), 3.41 (s, 3H, $OCH_3$), 3.26 (s, 3H, $OCH_3$), 3.14 (s, 3H, $OCH_3$). MS (neg. ion FAB): 1095 ($M^-$), 589, 321.

**Example 19**

Rapamycin 42-ester with (4-chlorophenylsulfonyl)carbamic acid

A solution of 400 mg of rapamycin in 10 mL ether/toluene (1:4) was treated at -10° under nitrogen with 100 mg of 4-chlorobenzenesulfonyl isocyanate in 1 mL toluene. The mixture was stirred at 0° under nitrogen for 3.5 hours, diluted with 120 mL of ethyl acetate, washed with saturated sodium bicarbonate solution, followed by a water wash, and dried with magnesium sulfate. The ethyl acetate solution was evaporated and the residue chromatographed on silica gel. Elution with ethyl acetate afforded 100 mg of the title compound as a white solid, mp 142- 146°. The title compound was isolated as a trihydrate.
$^1H$ NMR ($CDCl_3$, 400 MHz), δ 8.0 (d, J=13cps, 2H, aromatic protons meta to sulfonyl group), 7.53 (d, J=13 cps, 2H, aromatic protons ortho to sulfonyl group), 3.30 (s, 3H, $OCH_3$), 3.20 (s, 3H, $OCH_4$), 3.13 (s, 3H, $OCH_3$) ppm. MS (neg ion FAB): 1129 (MH-). IR KBr max 3430 (OH), 2930, 1740, 1730 (both C=O), 1640 (amide C=O), 1450, 1350 ($-SO_2-$), 1160 ($-SO_2-$), 1160 ($-SO_2-$), 1090, 985 $cm^{-1}$.

Anal. Calcd. for $C_{58}H_{83}ClN_2O_{16}S \cdot 3\ H_2O$: C 58.74; H 7.56; N 2.35
Found: C 58.92; H7.99; N 2.17

**Example 20**

Rapamycin 42-ester with (3-methylphenylsulfonyl)carbamic acid

A solution of 400 mg rapamycin in 10 mL ethyl acetate/ether (1/4) was treated at -10° under nitrogen with 127 mg of 3-methylphenylsulfonyl isocyanate in 2 mL ether. THe mixture was stirred at 0° under nitrogen for 4.5 hours, diluted with 120 mL ethyl acetate, washed with a saturated sodium bicarbonate solution, and dried with magnesium sulfate. The ethyl acetate solution was evaporated and the residue chromatographed on silica gel. Elution with ethyl acetate/n-hexane (1:1) afforded 270 mg of the title compound as a white powder, mp 116-120°.
$^1H$ NMR ($CDCl_3$, 400 MHz), δ 8.13 (d, J=13 cps, 1H, aromatic proton ortho to sulfonyl group), 7.53 (m, 1H, aromatic proton, para to sulfonyl group), 7.39 (m, 1H, aromatic protons para to methyl group), 7.33 (d, J=13 cps, 1H, aromatic ortho to methyl group), 3.23 (s, 3H, $OCH_3$), 3.18 (s 3H, $OCH_3$), 3.14 (s, 3H, $OCH_3$), 2.67 (s, 3H, aromatic methyl) ppm. MS (neg. ion FAB): 1109 (MH-). IR KBr max 3450 (OH), 2940, 1740, 1720 (C=O), 1640 (amide C=O), 1450, 1340 ($-SO_2-$), 1160 ($-SO_2-$), 990 $cm^{-1}$.

**Example 21**

Rapamyçin 31,42-diester with phenylsulfonylcarbamic acid

A solution of 200 mg of rapamycin 42-ester with phenylsulfonylcarbamic acid in 3 mL ethyl acetate/ether (1:1) was treated at 0° under nitrogen with 200 mg of benzenesulfonyl isocyanate in 0.5 mL of ether. After stirring at 0° under $N_2$ for 18 hours and at 22° under $N_2$ for 3 hours, the reaction mixture was diluted with 120 mL ethyl acetate, washed with a saturated sodium bicarbonate solution, and then washed with brine. The ethyl acetate solution was dried with magnesium sulfate and evaporated. The residue was chromatographed on silica gel. Elution with ethyl acetate afforded 75 mg of the title compound as a white foam, mp 104-108°.
NMR ($CDCl_3$, 400 MHz): δ 8.03 (m, 2H, aromatic), 7.55 (m, 3H, aromatic), 3.26 (s, 3H, $OCH_3$), 3.22 (s, 3H, $OCH_3$), 3. 13 (s, 3H, $OCH_3$) ppm. MS (neg. ion FAB): 1278 ($MH^-$). IR (KBr): 3340 (OH), 2930, 1730 & 1715 (C=O), 1630 (aromatic), 1445, 1290, 1160, 1095, 990 $cm^{-1}$.

## Claims

1. A compound of formula:

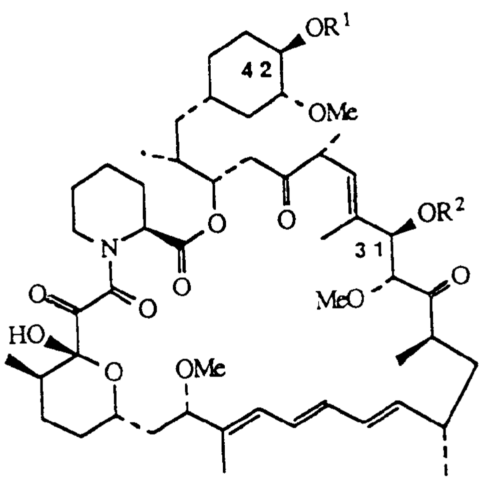

(I)

wherein $R^l$ and $R^2$ are each, independently, hydrogen, $-CONH(CR^3R^4)_n-X$ or $-CONHSO_2-Ar$;

$R^3$ and $R^4$ are each, independently, hydrogen, alkyl of l-6 carbon atoms, aralkyl of 7-l0 carbon atoms, cycloalkyl of 3-8 carbon atoms, halogen, or trifluoromethyl, providing that when more than one $R^3$ or $R^4$ group is present then they may each be the same or different;

X is hydrogen, lower alkyl of l-6 carbon atoms, cycloalkyl of 3-8 carbon atoms, trifluoromethyl, nitro, alkoxy of l-6 carbon atoms, carboalkoxy of 2-7 carbon atoms, aralkyl of 7-l0 carbon atoms, halo, dialkylamino of l-6 carbon atoms per alkyl group, thioalkyl of l-6 carbon atoms, or Y:

Y is a phenyl- group which may be optionally mono-, di-or tri- substituted with a group selected from alkyl of l-6 carbon atoms, aralkyl of 7-l0 carbon atoms, alkoxy of l-6 carbon atoms, cyano, halo, nitro, carbalkoxy of 2-7 carbon atoms, trifluoromethyl, dialkylamino of l-6 carbon atoms per alkyl group, or alkylthio of l-6 carbon atoms;

Ar is phenyl, naphthyl, pyridyl, quinolyl, isoquinolyl, quinoxalyl, thienyl, thionaphthyl, furyl, benzofuryl, benzodioxyl, benzoxazolyl, benzoisoxazolyl, or benzodioxolyl; wherein the Ar group may be optionally mono-, di- or tri-substituted with a group selected from alkyl of l-6 carbon atoms, arylalkyl of 7-l0 carbon atoms, alkoxy of l-6 carbon atoms, cyano, halo, nitro, carbalkoxy of 2-7 carbon atoms, trifluoromethyl, amino, dialkyl-amino of l-6 carbon atoms per alkyl group, alkylthio of l-6 carbon atoms, $-SO_3H$,$-PO_3H$, and $-CO_2H$; and

n = 0-5;

with the proviso that $R^l$ and $R^2$ are not both hydrogen and when n = 0, X is lower alkyl of l-6 carbon atoms, cycloalkyl of 3-8 carbon atoms, aralkyl of 7-l0 carbon atoms, or Y;

or a pharmaceutically acceptable salt thereof when X is dialkylamino of l-6 carbon atoms per alkyl group, when the Ar group contains a basic nitrogen, or when the Ar group is substituted by dialkylamino of l-6 carbon atoms per alkyl group, $-SO_3H$,$-PO_3H$, or $-CO_2H$.

**2.** A compound as claimed on Claim l where $R^l$ is hydrogen.

**3.** A compound as claimed on Claim l where $R^2$ is hydrogen.

**4.** A compound as claimed in any one of Claims l to 3 where n is 0 and X is Y.

**5.** A compound as claimed in any one of Claims l to 4 where Y is phenyl, 4-fluorophenyl, 2,4-difluorophenyl, 4-nitrophenyl, or 4-methylphenyl.

**6.** A compound as claimed in any one of Claims l to 3 wherein Ar is phenyl; the phenyl group being optionally mono-, di-, or tri-substituted or a pharmaceutically acceptable salt thereof.

**7.** A compound as claimed in Claim 6 wherein the substituent is selected from halogen and $C_l$-$C_4$ alkyl.

**8.** A compound as claimed in Claim l which is one of the following:
rapamycin 42-ester with (4-fluorophenyl)- carbamic acid;
rapamycin 42-ester with phenylcarbamic acid;
rapamycin -3l,42-diester with [4-(trifluoromethyl)-phenyl]carbamic acid;
rapamycin-42-ester with
[4-(trifluoromethyl)phenyl]carbamic acid;
rapamycin-3l,42-diester with (4-nitrophenyl)carbamic acid;
rapamycin-42-ester with (4-nitrophenyl)carbamic acid;
rapamycin-3l,42-diester with(4-methylphenyl)carbamic acid;
rapamycin-42-ester with (4-methylphenyl)carbamic acid;
rapamycin-42-ester with (2,4-difluorophenyl)carbamic acid;
rapamycin-3l,42-diester with
(2,4-difluorophenyl)carbamic acid;
rapamycin-3l,42-diester with phenylcarbamic acid;
rapamycin-3l,42-diester with (4-flurophenyl)carbamic acid;
rapamycin-3l-ester with (2,4-difluorophenyl)carbamic acid;
rapamycin 42-ester with phenylsulfonylcarbamic acid;
rapamycin-3l-ester with phenylsulfonylcarbamic acid;
rapamycin 42-ester with (4-chlorophenylsulfonyl)-carbamic acid;
rapamycin 42-ester with (3-methylphenylsulfonyl)-carbamic acid;

**9.** A modification of a compound as claimed in Claim I wherein $R^I$ is a silyl protecting group and $R^2$ is -CONH$(CR^3R^4)_n$-X or CONHSO$_2$Ar wherein n, X, $R^3$, $R^4$ and Ar are as defined in Claim I.

**10.** A compound as claimed in Claim 9 wherein the silyl protecting group is a tertbutyldimethylsilyl group.

**11.** A compound as claimed on Claim 9 which is rapamycin 42-tertbutyldimethylsilyl ether-3l-ester with phenylsulfonylcarbamic acid.

**12.** A compound as claimed in Claim 9 which is rapamycin-42-tert-butyldimethylsilyl ether-3l-ester with (2,4-difluorophenyl)carbamic acid.

**13.** A process for preparing a compound as claimed on Claim I which comprises carbamylating rapamycin; 42-$R^I$- substituted or 3l-$R^2$- substituted rapamycin in which $R^I$ and $R^2$ are as defined on Claim I or a 42 protected derivative of rapamycin using an isocyanate of formula

**O=N=C-Q**

wherein Q represents -$(CR^3R^4)_n$-X or -SO$_2$-Ar wherein n, $R^3$, $R^4$, X and Ar are as defined in Claim I and if required removing the 42 protecting group.

**14.** A process as claimed in Claim I3 wherein the protecting group is a tertbutyldimethylsilyl group.

**15.** A pharmaceutical composition comprising a compound as claimed on any one of Claims I to 8 and a pharmaceutically acceptable carrier.

**Claims for the following Contrating States: ES, GR**

**1.** A process for preparing a compound of formula

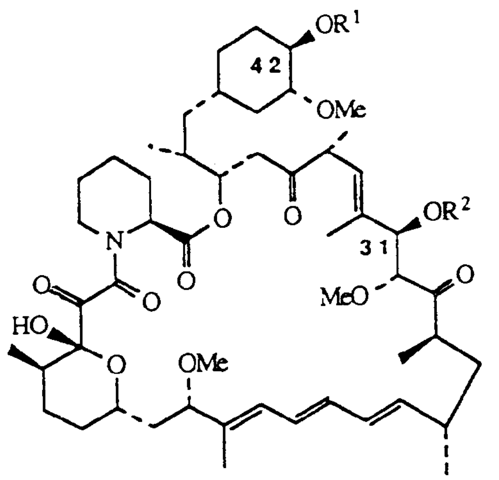

(I)

wherein $R^I$ and $R^2$ are each, independently, hydrogen, -CONH$(CR^3R^4)_n$-X or -CONHSO$_2$-Ar;

$R^3$ and $R^4$ are each, independently, hydrogen, alkyl of I-6 carbon atoms, aralkyl of 7-I0 carbon atoms, cycloalkyl of 3-8 carbon atoms, halogen, or trifluoromethyl, providing that when more than one $R^3$ or $R^4$ group is present when they may each be the same or different;

X is hydrogen, lower alkyl of I-6 carbon atoms, cycloalkyl of 3-8 carbon atoms, trifluoromethyl, nitro, alkoxy of I-6 carbon atoms, carboalkoxy of 2-7 carbon atoms, aralkyl of 7-I0 carbon atoms, halo, dialkylamino of I-6 carbon atoms per alkyl group, thioalkyl of I-6 carbon atoms, or Y:

Y is a phenyl group which may be optionally mono-, di-or tri- substituted with a group selected from alkyl of I-6 carbon atoms, aralkyl of 7-I0 carbon atoms, alkoxy of I-6 carbon atoms, cyano, halo, nitro, carbalkoxy of 2-7 carbon atoms, trifluoromethyl, dialkylamino of I-6 carbon atoms per alkyl group, or alkylthio of I-6 carbon atoms;

Ar is phenyl, naphthyl, pyridyl, quinolyl, isoquinolyl, quinoxalyl, thienyl, thionaphthyl, furyl, benzofuryl, benzodioxyl, benzoxazolyl, benzoisoxazolyl, or benzodioxolyl; wherein the Ar group may be optionally mono-, di- or tri-substituted with a group selected from alkyl of I-6 carbon atoms, arylalkyl of 7-I0 carbon atoms, alkoxy of I-6 carbon atoms, cyano, halo, nitro, carbalkoxy of 2-7 carbon atoms, trifluoromethyl, amino, dialkylamino of l-6 carbon atoms per alkyl group, alkylthio of l-6 carbon atoms, $-SO_3H$,$-PO_3H$, and $-CO_2H$; and

n = 0-5; with the provisos that

(i) $R^l$ and $R^2$ are not both hydrogen and

(ii) when n = 0, X is lower alkyl of l-6 carbon atoms, cycloalkyl of 3-8 carbon atoms, aralkyl of 7-l0 carbon atoms, or Y;

and

(iii) when $R^2$ is $-CONH(CR^3R^4)n-X$ or $CONHSO_2-Ar$ then $R^l$ may also represent a hydroxy protecting group or a pharmaceutically acceptable salt thereof when X is dialkylamino of l-6 carbon atoms per alkyl group, when the Ar group contains a basic nitrogen, or when the Ar group is substituted by dialkylamino of l-6 carbon atoms per alkyl group, $-SO_3H$,$-PO_3H$, or $-CO_2H$; which comprises carbamylating rapamycin;

42-$R^l$- substituted or 3l-$R^2$- substituted rapamycin on which $R^l$ and $R^2$ are as defined in Claim l or a 42 protected derivative of rapamycin using an isocyanate of formula

**O=N=C-Q**

wherein Q represents $-(CR^3R^4)_n-X$ or $-SO_2-Ar$ wherein n, $R^3$, $R^4$, X and Ar are as defined in Claim l and if desired removing the 42 protecting group.

**2.** A process as claimed in Claim l where $R^l$ is hydrogen.

**3.** A process as claimed in Claim l where $R^2$ is hydrogen.

**4.** A process as claimed in any one of Claims l to 3 where n is 0 and X is Y.

**5.** A process as claimed on any one of Claims l to 4 where Y is phenyl, 4-fluorophenyl, 2,4-difluorophenyl, 4-nitrophenyl, or 4-methylphenyl.

**6.** A process as claimed in any one of Claims l to 3 wherein Ar is phenyl; the phenyl group being optionally mono-, di-, or tri-substituted or a pharmaceutically acceptable salt thereof.

**7.** A process as claimed in Claim 6 wherein the substituent is selected from halogen and $C_l$-$C_4$ alkyl.

**8.** A process as claimed on Claim l wherein $R^l$ is a silyl protecting group.

**9.** A process as claimed in Claim l in which the product is one of the following:
rapamycin 42-ester with (4-fluorophenyl)carbamic acid;
rapamycin 42-ester with phenylcarbamic acid;
rapamycin -3l,42-diester with [4-(trifluoromethyl)-phenyl]carbamic acid;
rapamycin-42-ester with
[4-(trifluoromethyl)phenyl]carbamic acid;
rapamycin-3l,42-diester with (4-nitrophenyl)carbamic acid;
rapamycin-42-ester with (4-nitrophenyl)carbamic acid;
rapamycin-3l,42-diester with(4-methylphenyl)carbamic acid;
rapamycin-42-ester with (4-methylphenyl)carbamic acid;
rapamycin-42-ester with (2,4-difluorophenyl)carbamic acid;
rapamycin-3l,42-diester with
(2,4-difluorophenyl)carbamic acid;
rapamycin-3l,42-diester with phenylcarbamic acid;
rapamycin-3l,42-diester with (4-flurophenyl)carbamic acid;
rapamycin-3l-ester with (2,4-difluorophenyl)carbamic acid;
rapamycin 42-ester with phenylsulfonylcarbamic acid;
rapamycin-3l-ester with phenylsulfonylcarbamic acid;
rapamycin 42-ester with (4-chlorophenylsulfonyl)-carbamic acid;
rapamycin 42-ester with (3-methylphenylsulfonyl)-carbamic acid;
rapamycin 42-tertbutyldimethylsilyl ether-3l-ester with phenylsulfonylcarbamic acid;
and
rapamycin-42-tert-butyldimethylsilyl ether-3l-ester with (2,4-difluorophenyl)carbamic acid.

**10.** A process for preparing a pharmaceutical composition which comprises bringing a compound of formula

I as defined in any one of Claims l to 9 providing that $R^l$ is not a protecting group, into association with a pharmaceutically acceptable carrier.